# EUROPEAN PATENT APPLICATION

(11) **EP 4 190 358 A1**
(43) Date of publication of application: **07.06.2023**
(21) Application number: 21306709.3
(22) Date of filing: 05.12.2021
(51) Int. Cl.: A61K 47/42, A61K 38/16, C07K 14/195

(54) **POLYPEPTIDE AS CARRIER FOR INTESTINAL BARRIER CROSSING**

(71) Applicant: AFFILOGIC, 44200 Nantes (FR)
(72) Inventor: The designation of the inventor has not yet been filed
(74) Representative: Flesselles, Bruno F.G.

(57) **Abstract**

The invention relates to a polypeptide comprising a variant of a protein of the Sac7d family that specifically binds to the human leptin receptor and is able to trigger the crossing of the intestinal barrier, in particular of biologicals associated with it.

## Description

Increasing understanding of the disease mechanisms and of their pathogenesis at the molecular level has fueled the development of targeted therapies. In that field, biologics have been fully integrated in clinical practices, and are expected to represent a growing proportion of the therapeutic developments due to their potential at providing higher specificity compared with conventional small molecules.

Two main challenges remain to be addressed to close the gap between the development of small molecule and biologics, which are i) cost of development that are several fold higher for biologics and ii) the administration of the biologics limited almost exclusively to the parenteral routes.

Feedbacks from patients rank oral administration as the most preferred route of administration, which correlates with the higher compliance for oral treatments than parenteral drugs. Despite the preference for oral treatments, the development of oral biologics have been limited due to a lack of stability of the biologics in the gastric environment and the poor passive diffusion of large molecules through biological barriers such as the intestinal barrier. Nature engineered receptor-mediated active translocation mechanisms for the translocation of large biomolecules through biological barriers. Hijacking of these translocation mechanisms has been explored for the transport of drugs through the blood brain barrier, using ligand binding to receptors naturally mediating the transport of biomolecules in the brain such as TfR, LRP1 and the insulin receptor as Trojan horse delivery system.

The development of a similar strategy for the transport across the intestinal barrier would allow the systemic delivery of a cargo molecule upon oral administration. This requires a technology that can remain stable in the lumen of the intestine. Nanofitins are small alternative scaffold proteins derived from the natural hyperthermostable protein sac7d, which can be engineered for high specificity and high affinity toward a given target while retaining the astonishing stability of their parental protein, including resistance to protease degradation.

Leptin is a peptide hormone secreted mainly by adipocytes and tissues such as stomach, with multiple physiological effects. It primarily acts on the central nervous system, to regulate energy homeostasis and neuroendocrine function, and plays a role in diverse functions in the digestive tract, such as tissue healing or glucose metabolism. Therefore, this pluripotent hormone requires interactions with its cell surface receptor, human leptin receptor (human LepR or hLepR, UniProtKB - P48357), which is consequently present at various barriers such as blood brain barrier or intestinal barrier. There, the leptin receptor is responsible for receptor-mediated active translocation of its natural ligand, the leptin, as described at the blood brain barrier and at the intestinal barrier.

### SUMMARY OF THE INVENTION

In a first aspect, the invention relates to a polypeptide comprising a variant of a member of the Sac7d family binding to the human Leptin receptor (as well as to the mouse and pig leptin receptors), wherein the variant comprises from 4 to 20 mutated residues in the interface of binding of the member of the Sac7d family to its natural ligand, and wherein said variant comprises from 5 to 15 mutations with the numbering corresponding to Sac7d (SEQ ID NO: 1) residues. In particular, the variant comprises from 5 to 14 or from 5 to 13 mutated residues in the interface of binding of the member of the Sac7d family to its natural ligand. In other embodiments, the variant comprises from 6 to 15, or from 6 to 14 or from 6 to 13 mutated residues. In some embodiments, the variant contains exactly 15 mutated residues (not considering deletions at the N- or C-terminus). In some embodiments, the variant contains exactly 14 mutated residues (not considering deletions at the N- or C-terminus). In some embodiments, the variant contains exactly 13 mutated residues (not considering deletions at the N- or C-terminus). In some embodiments, the variant contains exactly 12 mutated residues (not considering deletions at the N- or C-terminus).

In particular, the polypeptide further comprises W24Y, T33W and A44H mutations, with the numbering corresponding to Sac7d (SEQ ID NO: 1) residues.

As shown in the examples, these mutations are the ones that are present in all members of cluster A, which regroups identified binders that present homology in the mutations present in the binding site (same amino acids, or same type (with regards to size, hydrophobicity, polarity...) of amino acids). Furthermore, the examples show that these variants bind to the human, mouse and pig leptin receptor, thereby making it very suitable for pharmacological development. The fact that the variants belonging to cluster A bind to the leptin receptor and don't compete with the leptin is also advantageous with regards to use in a pharmacological context as this may reduce side effects that could be observed if leptin binding to its receptor was abolished or diminished. It is also to be noted that, even though it is known that variants of the Sac7d family can pass the intestinal barrier (WO2016062874 and US 20180085427, and as shown in the examples that confirm that a small part of the non-specific variants cross the barrier), the amount of the specific variants herein disclosed that cross the intestinal barrier is much higher than the one of non-specific variants or of binders of the leptin receptor belonging to another cluster. This strongly suggests an active passage for the binders of cluster A *vs* a passive passage for other Sac7d variants. It is indeed been shown that the binding to the leptin receptor at the surface of intestinal cells triggers transport of the variant (alone or bound to another polypeptide) to the other side of the intestine.

In particular, the mutated residues in the interface of binding of the member of the Sac7d family to its natural ligand are selected from the group consisting of V2, K3, K5, K7, Y8, K9, G10, E14, T17, K21, K22, W24, V26, G27, K28, M29, S31, T33, D36, N37, G38, K39, T40, A44, S46, E47, K48, D49, A50 and P51 of Sac7d (SEQ ID NO: 1).

In some embodiments, the mutated residues are on K7, Y8, K9, K21, K22, W24, V26, M29, S31, T33, T40, R42, A44 and S46 of Sac7d (SEQ ID NO: 1).

In some embodiments, the mutated residues are on K7, Y8, K9, K21, K22, W24, V26, M29, S31, T33, R42 and A44 of Sac7d (SEQ ID NO: 1).

In some embodiments, the polypeptide further comprises at least one mutation selected from D16E, N37Q and M57L, with the numbering corresponding to Sac7d (SEQ ID NO: 1) residues.

In particular the member of the Sac7d family is selected from the group consisting of Sac7d from *Sulfolobus acidocaldarius,* Sac7e from *Sulfolobus acidocaldarius,* SSo7d from *Sulfolobus solfataricus,* Ssh7b from *Sulfolobus shibatae,* Ssh7a from *Sulfolobus shibatae,* DBP7 from *Sulfolobus tokodaii,* Sis7a from *Sulfolobus islandicus,* Mse7 from *Metallosphaera sedula,* Mcu7 from *Metallosphaera cuprina,* Aho7a from *Acidianus hospitalis,* Aho7b from *Acidianus hospitalis,* Aho7c from *Acidianus hospitalis* and Sto7 from *Sulfurisphaera tokodaii.*

Based on a consensus sequence for the Sac7d family (SEQ ID NO: 16), the variants comprise SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28 or SEQ ID NO: 29, or amino acids 5-56, 5-57, 5-58, 5-59, 5-60 or 5-61 of these sequences.

In some embodiments, the polypeptide is based on Sac7d and comprises SEQ ID NO: 35, SEQ ID NO: 36, SEQ ID NO: 37, SEQ ID NO: 38, SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 45, SEQ ID NO: 47, SEQ ID NO: 48 or SEQ ID NO: 49, or amino acids 1-54 of these sequences.

These sequences differ by (1) the specification of preferred mutations and (2) the presence or absence of specific mutations outside the binding site and which are favorable for stability and/or industrial production.

In some embodiments, the polypeptide is based on Aho7c and comprises SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, or SEQ ID NO: 64 or amino acids 1-54 of these sequences.

Variants based on the consensus sequence for Sac7d and Aho7c (depicted by SEQ ID NO: 65) comprises SEQ ID NO: 30, SEQ ID NO: 31, SEQ ID NO: 32, SEQ ID NO: 33 or SEQ ID NO: 34, or amino acids 1-54 of these sequences.

In some embodiments, the polypeptide is based on Sso7d and comprises SEQ ID NO: 66, SEQ ID NO: 67, SEQ ID NO: 68, SEQ ID NO: 69 or SEQ ID NO: 70 or amino acids 1-53 or 1-54 of these sequences.

In some embodiments, the polypeptide may comprise amino acids 1-55, 1-56, 1-57, 1-58 or 1-59 of the above mentioned sequences.

In some embodiments, the polypeptide consists in the variant of a member of the Sac7d family binding to the human leptin receptor.

In some embodiments, the variant of a member of the Sac7d family binding to the human leptin receptor is conjugated to an organic molecule.

In some embodiments, the variant of a member of the Sac7d family binding to the human leptin receptor is conjugated to another polypeptide, in particular comprising another variant of a protein of the Sac7d family. In some embodiments, the variant of a member of the Sac7d family binding to the human leptin receptor is conjugated to another variant of a protein of the Sac7d family.

The invention also relates to a nucleic acid molecule coding for the polypeptide as described, an expression vector comprising such nucleic acid molecule (and including elements allowing transcription in a host cell), and to a host cell comprising the nucleic acid molecule or the expression vector.

The invention also relates to a pharmaceutical composition comprising the polypeptide as disclosed, the nucleic acid as disclosed, the expression vector as disclosed, or the host cell as disclosed, and a pharmaceutically acceptable carrier.

The invention also relates to a method for producing the polypeptide as disclosed, comprising
a. Culturing a cell culture wherein the cells have been transformed by the expression vector as disclosed,
   And
b. Recovering the polypeptide.

The invention also relates to the polypeptide or the nucleic acid as disclosed for use thereof as a medicament.

The invention also relates to the leptin receptor binding variant fused to an active molecule, in particular another polypeptide, the nucleic acid coding for it, the expression vector, or the cell as disclosed for use for the treatment of prevention of a disease, wherein the variant is associated with a substance active for treatment or prevention of the disease. This substance may be designated as a therapeutically active substance.

The invention also relates to a method of treating a subject having or at risk of developing a given disease, comprising administering to the subject a composition comprising an effective amount of the LepR-binding variant associated or bound to a substance therapeutically active for treating or preventing the given disease.

### DETAILED DESCRIPTION OF THE INVENTION

In particular, the invention relates to a polypeptide comprising a variant of the Sac7d protein or of a protein of the Sac7d family that specifically binds to the human leptin receptor (referred to as P48357 in the UNIPROT database). In particular, the variant binds to the human, pig and mouse leptin receptor. It also preferably allows crossing of the intestinal barrier, when bound to another polypeptide, in particular another variant of a protein of the Sac7d family. Such variant is useful for being fused, bound or associated to a therapeutically active substance to allow oral or rectal administration of such therapeutically active substance, crossing the intestinal barrier and action of the therapeutically active substance, in particular locally in the intestine, or in other organs of the body, after passage through the systemic circulation.

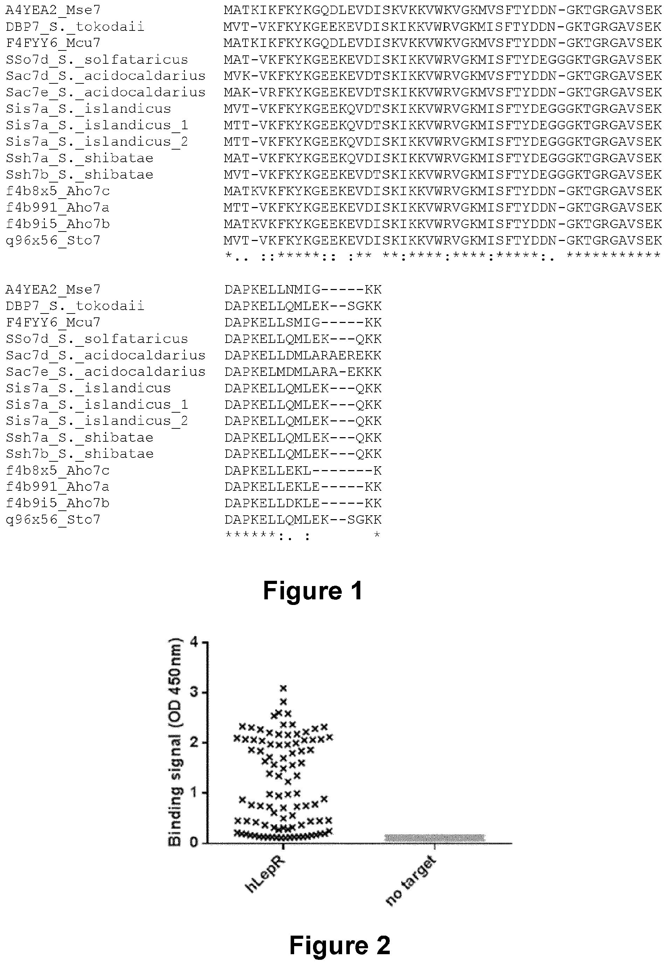

In preferred embodiments, such variant comprises SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, or SEQ ID NO: 29. These sequences are based on the sequence consensus for the Sac7d family and have been obtained following the teachings of WO 2012/150314 which shows that it is possible to perform portability of the mutations from one protein of the Sac7d family to another one, these proteins presenting a similar structure and binding site as well as similar amino acid sequences. SEQ ID NO: 22-25 are the consensus sequence with a D at position 17, whereas SEQ ID NO: 26-29 present a E at position 17. Such position 17 corresponds to position 16 of SEQ ID NO: 1 as seen on the sequence alignment of Figure 1. One, two or three of first amino acids of these sequences may be omitted, as may be some or all of the last 12.

In some embodiments said variant is based on the Sac7d protein, and comprises SEQ ID NO: 35, SEQ ID NO: 36, SEQ, ID NO: 37, SEQ ID NO: 38, SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46, SEQ ID NO: 47, SEQ ID NO: 18, or SEQ ID NO: 49, or amino acids 1-54, 1-55, 1-56, 1-57, 1-58, 1-59, 1-60, 1-61, 1-62 or 1-63 of these sequences. SEQ ID NO: 41-44 represent the variant with the native amino acids D, N and M at positions 16, 37 and 57 of SEQ ID NO: 1 (the starting M of SEQ ID NO: 1 being omitted in these sequences), whereas SEQ ID NO: 45-49 represent the variant with modified amino acids E, Q and L at these positions. SEQ ID NO: 35-39 represent consensus sequences for these positions.

In some embodiments, said variant is based on the Sso7d protein (SEQ ID NO: 2), and comprises SEQ ID NO: 67, SEQ ID NO: 68, SEQ ID NO: 69 or SEQ ID NO: 70, or amino acids 1-53, 1-54, 1-55, 1-56 of these sequences. In these sequences, the first three amino acids of SEQ ID NO: 2 have been omitted.

In some embodiments said variant is based on the Aho7c protein (SEQ ID NO: 14), and comprises SEQ ID NO: 50, SEQ ID NO: 51, SEQ, ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, or SEQ ID NO: 64, or amino acids 1-54, 1-55, 1-56 or 1-57 of these sequences. SEQ ID NO: 55-59 represent the variant with the native amino acids D and N at positions 17 and 38 of SEQ ID NO: 14 (the starting two amino acids MA of SEQ ID NO: 14 being omitted in these sequences), whereas SEQ ID NO: 60-64 represent the variant with modified amino acids E and Q at these positions. SEQ ID NO: 50-54 represent consensus sequences for these positions.

Variants based on the consensus sequence for Sac7d and Aho7c are represented by SEQ ID NO: 30, SEQ ID NO: 31, SEQ ID NO: 32, SEQ ID NO: 33 and SEQ ID NO: 34.

It is to be noted that the sequences indicated above don't comprise a methionine at its N-terminal, but that it is also possible to perform the invention with a methionine at the N-terminal end of each sequence.

Such variant can be fused to a biomolecule and/or conjugated to a small chemical entity and be used by oral or rectal administration to allow crossing of the intestinal barrier of the molecule, thus increasing efficacy or bioavailability, or allowing use of a lesser amount. Typical molecules that can be conjugated are small chemical molecules (e.g. cytotoxic agents against cancer such as auristatin or doxorubicin), peptides (insulin analogs such as GLP-1), small protein scaffolds such as Sac7d from *Sulfolobus acidocaldarius* (and analogs such as Aho7c, or Sso7d) derivatives, antibody mimetics such as affibodies, affilins, affimers, alphabodies, anticalins, avimers, DARPins, fynomers, Kunits domain peptides, monobodies, Z domain of Protein A, Gamma B crystalline, ubiquitin, cystatin, lipocalin, A domain of a membrane receptor, ankyrin repeat motive, SH3 domain of Fyn, Kunits domain of protease inhibitors, the 10th type III domain of fibronectin, 3-or 4-helix bundle proteins, an armadillo repeat domain, a leucine-rich repeat domain, a PDZ domain, a SUMO or SUMO-like domain, an immunoglobulin-like domain, phosphotyrosine-binding domain, pleckstrin homology domain, src homology 2 domain or synthetic peptide ligands, antibody fragments (including Fabs, VHH, ScFv), enzymes, therapeutic nucleic acids such as Antisense Oligonucleotides, siRNAs, shRNAs. Larger polypeptides can also be fused to the variant herein disclosed.

All these sequences describe specific variants based on proteins of the Sac7d family. As explained below, it is possible, using the alignment of Figure 1, to design variants of other proteins of the family.

A sequence that is consensus for proteins Sac7d and Aho7c is SEQ ID NO: 65. SEQ ID NO: 30, SEQ ID NO: 31, SEQ ID NO: 32 and SEQ ID NO: 34 are variants based on this consensus sequence.

In case of discrepancy between the specification and the sequence listing, the sequences mentioned in the specification are preponderant.

As indicated above, the specification specifically discloses variants of Sac7d (SEQ ID NO: 1) Aho7c (SEQ ID NO: 14), Sso7d (SEQ ID NO: 2), but the teachings are applicable to the other proteins of the Sac7d family, in particular Sto7 (SEQ ID NO: 15) which is very similar to Sac7d and Aho7c. The teachings are also applicable to other OB-fold domains as disclosed in WO2007139397. The invention is also applicable to SH3 domains, a small protein domain of about 60 amino acid residues, initially, described as a conserved sequence in the viral adaptor protein v-Crk and described under PF00018 in the PFAM database. The SH3 domain has a characteristic beta-barrel fold that consists of five or six β-strands arranged as two tightly packed anti-parallel β sheets. The linker regions may contain short helices. It is to be noted that OB-fold and SH3 domains share homology and that, in view of the knowledge of the sequence and structure of these domains, it is possible to determine, in any of OB-fold or SH3 domain, to which amino acids correspond the amino acids as disclosed below for Sac7d.

In a specific embodiment, the polypeptide consists in the variant of a protein of the Sac7d family binding to the human leptin receptor.

In a specific embodiment, the variant of a protein of the Sac7d family binding to the human leptin receptor is linked or fused to another protein or polypeptide. In particular, the other protein or polypeptide may be the same or another variant of a protein of the Sac7d family, binding to another target. In this embodiment, it is preferred when the other variant of the Sac7d family binds to a target associated with a disease, so the other variant is useful for treating or preventing the disease. In some embodiment, the other variant of the Sac7d family binds to human serum albumin. In some embodiments, the leptin receptor binding variant is bound to (at least) two other variants of the Sac7d family.

In specific embodiments, the variant is present in a polypeptide and is thus covalently linked by amine bonds to other proteins or polypeptides presenting a biological interest.

In an embodiment, the polypeptide is conjugated to an organic molecule that presents some therapeutic activity.

The invention also pertains to a genetic construct comprising a DNA sequence coding for the polypeptide described herein, to a vector comprising such genetic construct, and to a host cell comprising the genetic construct in its genome.

The invention also pertains to a method for producing the polypeptide herein disclosed, comprising the steps consisting of
a. Culturing a cell culture wherein the cells have been transformed by a genetic construct as disclosed,
   and
b. Recovering the polypeptide.

The invention also relates to a polypeptide herein disclosed for use thereof as a medicament.

The invention also relates to a method for treating a subject in need thereof comprising administering a therapeutic amount of a variant binding to leptin receptor herein disclosed to the subject, wherein the variant is fused to a peptide or protein with therapeutic activity against a disease as a medicament.

The invention also relates to a composition containing a variant binding to leptin receptor herein disclosed to the subject, wherein the variant is fused to a peptide or protein with therapeutic activity against a given disease and another agent for simultaneous, separate or sequential (spread out over time) use in the treatment of the disease. Such other agent is selected among agents already known for treating the given disease.

It is particularly adapted when the disease is as disclosed below.

The invention also relates to these variants in therapeutic, diagnostic or purification uses. The invention also relates to compositions, in particular oral compositions, containing the polypeptide or the variants. Indeed, and even for treating systemic diseases (not localized in the gastrointestinal system), oral administration is preferred, as far as possible, to other modes of administration (and in particular injections), which can require the involvement of members of the care staff. In this embodiment, the composition also preferably contains a pharmaceutically acceptable excipient (such as a dye, a sweetener, a texture agent) that can be used for such an oral administration

It is reminded that the sequence of Sac7d is: 
MVKVKFKYKGEEKEVDTSKIKKVWRVGKMVSFTYDDNGKTGRGAVSEKDAPKEL
LDMLARAEREKK (SEQ ID NO: 1).
 The sequence of Sso7d is 
MATVKFKYKGEEKEVDISKIKKVWRVGKMISFTYDEGGGKTGRGAVSEKDAP
KELLQMLEKQKK (SEQ ID NO: 2).
 The sequence of Sso7d is

MATKVKFKYKGEEKEVDISKIKKVWRVGKMISFTYDDNGKTGRGAVSEKDAP
KELLEKLK (SEQ ID NO: 14).

The variants binding to the human leptin receptor herein disclosed contain mutations at positions corresponding to the positions 7, 8, 9, 21, 22, 24, 26, 29, 31, 33, 40, 42, 44 and/or 46 of the Sac7d sequence. It is however to be noted that the threonine at position 40 may be maintained in the variants and/or the serine at position 46. However, the variants binding to human leptin receptor herein disclosed contain at least 4, more preferably at least 5, more preferably at least 6, more preferably at least 7, more preferably at least 8, more preferably at least 9, more preferably at least 10, more preferably at least 11, more preferably at least 12 mutated amino acids as compared to SEQ ID NO: 1 (i.e. amino acids that are different than the corresponding ones of SEQ ID NO: 1, or to the sequence of the protein of the Sac7d family from which they are issued). The generally present at most 20, more preferably at most 18, more preferably at most 16, 15, 14 or 13 mutated amino acids as compared to SEQ ID NO: 1 (or to the sequence of the protein of the Sac7d family from which they are issued).

### The Sac7d protein family

The Sac7d family is defined as relating to the Sac7d protein and corresponds to a family of 7 kDa DNA-binding proteins isolated from extremophilic bacteria. It is herein disclosed as a representative species of OB-fold domains, that is preferably used in the context of the invention. Since SH3 domains share homology with OB-fold domains, the teachings pertaining to Sac7d are also applicable to SH3 scaffolds.

These proteins and this family are in particular described in WO 2008/068637. Thus, within the context of the present invention, a protein belongs to the Sac7d family when it has one of the sequences SEQ ID NO: 1 to SEQ ID NO: 15, or when it has a sequence corresponding to the sequence SEQ ID NO: 16, which is a consensus sequence (obtained from SEQ ID NO: 1 to SEQ ID NO: 9 and SEQ ID NO: 12 to SEQ ID NO: 15. In this consensus sequence, a dash - indicates no amino acid, the proteins not having all the same size). This Sac7d family comprises in particular the Sac7d or Sac7e proteins derived from *Sulfolobus acidocaldarius,* the Sso7d protein derived from *Sulfolobus solfataricus,* the DBP 7 also called Sto7 protein derived from *Sulfolobus tokodaii,* the Ssh7b protein derived from *Sulfolobus shibatae,* the Ssh7a protein derived from *Sulfolobus shibatae,* Mse7 derived from *Metallosphaera sedula,* Mcu7 derived from *Metallosphaera cuprina,* Aho7a or Aho7b or Aho7c derived from *Acidianus hospitalis,* Sis7a or Sis7b derived from *Sulfolobus islandicus* and the p7ss protein derived from *Sulfolobus solfataricus.* In view of the broad similarity of the sequences of the proteins of the Sac7d family, it is directly possible and easy to identify the amino acids of another protein than Sac7d that correspond to given amino acids of Sac7d. In particular, one can also use the teachings of WO 2008/068637 that shows (in the figures) and explains (in the specification) that such proteins are superimposable. The contents of this document are incorporated herein by reference, in particular with regards to the description of methods for aligning and superimposing various OB-fold proteins. As indicated above, it is useful to use the numeration of the amino acids from the Sac7d protein for designating a specific amino acid, and the equivalent amino acids can be obtained from Figure 1.

WO 2012/150314 shows that the mutations from one protein of the Sac7d family can be carried to another protein of the same family. This portability amounts to creating a mutant of another protein of the Sac7d family, starting from a mutant of one protein of said family. The first mutant may have been obtained in particular by carrying out the process of WO 2008/068637. Consequently, it is possible, using in particular the teachings of WO 2012/150314 and of figure 1, to obtain a mutant of any protein of the Sac7d family, starting from a mutant of any other protein of such family. As an illustration, for a mutant of Sac7d, one can introduce the mutated amino acids of the Sac7d mutant within the scaffold of another protein, using the sequence alignment of figure 1. As an illustration, variants of Sso7d, obtained from the Sac7d variants herein disclosed, are described as SEQ ID NO: 66, SEQ ID NO: 67, SEQ ID NO: 68, SEQ ID NO: 69 and SEQ ID NO: 70.

Using the consensus sequence of the Sac7d family, variants are depicted by SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 28, SEQ ID NO: 28 or SEQ ID NO: 28.

The number of mutated residues introduced within a wild-type protein sequence to obtain a variant is preferably between 4 and 25, or more specifically between 4 and 22 or between 4 and 20. It is thus possible to obtain variants preferably having at least 4, more preferably at least 5, more preferably at least 6, more preferably at least 7 or 8, even more preferably at least 10, but generally less than 25, more preferably less than 22, even more preferably less than 20 or less than 15 or 14 substituted amino acids compared with the wild-type OB-fold protein (or domain). It is to be noted that all and any ranges are considered herein, (such as 5-20 or 7-25 and so forth). Particularly preferred ranges are 4-20, 4-17 and 6-17, 4-14 and 6-14.

It is preferred when 7, 8, 9, 10, 11, 12, 13 or 14 amino acids are mutated in the binding site of the OB-fold domain, relative to the wild-type OB-fold domain. These mutations are thus preferably introduced in the amino acids corresponding to V2, K3, K5, K7, Y8, K9, G10, E11, K13, E14, T17, K21, K22, W24, V26, G27, K28, M29, S31, T33, Y34, D36, N37, G38, K39, T40, R42, A44, S46, E47, K48, D49, A50 and P51 of Sac7d (SEQ ID NO: 1), more specifically K7, Y8, K9, K21, K22, W24, V26, G27, K28, M29, S31, T33, T40, R42, A44 and S46 or K7, Y8, K9, K21, K22, W24, V26, G27, K28, M29, S31, T33, R42 and A44. As indicated, the variants contain the mutations W24Y, T33W and A44H. Other mutations (D16E, N37Q and M57L) can also be performed. All amino acid numbers are with reference to SEQ ID NO: 1.

In particular, it is interesting when the number of mutated amino acids is between 7 and 14 (limits included). In particular, the variants can also comprise amino acid insertions as indicated in WO 2008/068637.

As indicated, proteins of the Sac7d family are Sac7d or Sac7e derived from *Sulfolobus acidocaldarius,* Sso7d derived from *Sulfolobus solfataricus,* DBP 7 also called Sto7 derived from *Sulfolobus tokodaii,* Ssh7b derived from *Sulfolobus shibatae,* Ssh7a derived from *Sulfolobus shibatae,* Mse7 derived from *Metallosphaera sedula,* Mcu7 derived from *Metallosphaera cuprina,* Aho7a or Aho7b or Aho7c derived from *Acidianus hospitalis,* Sis7a or Sis7b derived from *Sulfolobus islandicus* and p7ss derived from *Sulfolobus solfataricus.* The various sequences of the Sac7d, Sso7d, Sac7e, Ssh7b, Ssh7a, DBP7, Sis7a (3 alleles), Mse7, Mcu7, Aho7a, Aho7b, Aho7c and Sto7 proteins are represented by SEQ ID NO: 1 to SEQ ID NO: 15 respectively.

A variant of a protein of this Sac7d family may be called a nanofitin. The invention is thus preferentially implemented on variants of the proteins represented by SEQ ID NO: 1 to SEQ ID NO: 15, or a protein having a sequence that reads on SEQ ID NO: 16 (consensus sequence), in particular on variants of Sac7d.

### Link of Sac7d protein with OB-fold proteins and SH3 domains

The OB-fold proteins are known in the art. They are in particular described in the documents cited above, and also in Arcus (Curr Opin Struct Biol. 2002 Dec; 12(6):794-801). OB-fold is in the form of a cylinder having five beta (β) sheets. Most OB-fold proteins use the same binding interface of their natural ligand, which may be an oligosaccharide, an oligonucleotide, a protein, a metal ion or a catalytic substrate. This binding interface comprises mainly the residues located in the beta sheets. Certain residues located in the loops may also be involved in the binding of an OB-fold protein with its natural ligand. Thus, applications WO 2007/139397 and WO 2008/068637 and the Arcus document (2002, op. cit.) describe the OB-fold-protein domains for binding with their natural ligand.

In particular, document WO 2008/068637 describes precisely how to identify the binding domain of an OB-fold protein. By superimposing several sequences and 3D structures of proteins having OB-fold domains, using WU-Blast2 (Lopez et al., 2003, Nucleic Acids Res 31, 3795-3798), T-COFFEE (Notredame et al., 2000, J Mol Biol 302, 205-217) or DALI lite (Holm and Park, 2000, Bioinformatics 16, 566-567), it is possible to identify the positions of the binding domains and in particular the amino acids which can be modified. Taking the sequence of Sac7d (SEQ ID NO: 1) as a reference, these are the residues V2, K3, K5, K7, Y8, K9, G10, E11, K13, E14, T17, K21, K22, W24, V26, G27, K28, M29, S31, T33, Y34, D35, D36, N37, G38, K39, T40, G41, R42, A44, S46, E47, K48, D49, A50 and P51.

WO 2008/068637 describes that it is possible to perform a superimposition of 3D structures of OB-fold proteins or domains (10 domains were used in this application, including Sac7d), using the DALI website (http://www.ebi.ac.uk/dali/interactive.html)(Holm and Sander, 1998, Nucleic Acids Res 26, 316-319). Thus, it is easy to identify, for any OB-fold protein (or any OB-fold domain), the amino acids involved in the binding site and corresponding to the Sac7d amino acids mentioned above. Consequently, providing the amino acids that can be mutated in one of these proteins makes it possible to identify the corresponding amino acids for any other OB-fold domain.

It is also to be noted that OB-fold domains resemble SH3 domains and that it is also possible to identify equivalents of amino acids of Sac7d in SH3 domains.

### Example of OB-fold or SH3 domain

Non-limitative examples of OB-fold proteins which can be used according to the invention are Sac7d, Sso7d, the N-terminal domain of SEB (Papageorgiou et al., 1998), the chain A of the Shiga-like toxin IIe (PDB 2bosa), the human Neutrophil Activatin Peptide-2 (NAP-2, PDB 1tvxA), the Molybdenum Binding Protein (modg) of Azotobacter vinelandii (PDB 1h9j), the N-terminal domain of SPE-C (Roussel et al., 1997), the B5 subunit of E. coli Shiga-like toxin (Kitov et al., 2000), Cdc13 (Mitton-Fry et al., 2002), the cold-shock DNA-binding domain of the human Y-box protein YB-1 (Kloks et al., 2002), the E. coli inorganic pyrophosphatase EPPase (Samygina et al., 2001), or any of the proteins listed in Table 3 of the article by (Arcus, 2002), such as 1krs (Lysyl-tRNA synthetase LysS, E.coli), 1c0aA (Asp- tRNA synthetase, E.coli), 1b8aA (Asp- tRNA synthetase, P. kodakaraensis), 1lylA (Lysyl-tRNA synthetase LysU, E.coli), 1quqA (Replication protein A, 32kDa subunit, Human), 1quqB (Replication protein A, 14kDa subunit, Human), 1jmcA (Replication protein A, 70kDa subunit (RPA70) fragment, Human), 1otc (Telomere-end-binding protein, O. nova), 3ullA (Mitochondrial ssDNA-binding protein, Human), 1prtF (Pertussis toxin S5 subunit, B. pertussis), 1bcpD (Pertussis toxin S5 subunit (ATP bound), B. pertussis), 3chbD (Cholera Toxin, V. cholerae), 1tiiD (Heat-labile toxin, E. coli), 2bosA (Verotoxin-1/Shiga toxin, B-pentamer, E. coli), 1br9 (TIMP-2, Human), 1an8 (Superantigen SPE-C, S. pyogenes), 3seb (Superantigen SPE, S. aureus), 1aw7A (Toxic shock syndrome toxin, S. aureus), 1jmc (Major cold-shock protein, E.coli), 1bkb (Initiation translation factor 5a, P. aerophylum), 1sro (S1 RNA-binding domain of PNPase, E.coli), 1d7qA (Initiation translation factor 1, elF1a, Human), 1ah9 (Initiation translation factor 1, IF1, E.coli), 1b9mA (Mo-dependent transcriptional regulator ModE, E.coli), 1ckmA (RNA guanylyltransferase, Chlorella virus, PBCV-1), 1a0i (ATP-dependent DNA ligase, Bacteriophage T7), 1snc (Staphylococcal nuclease, S. aureus),1hjp (DNA helicase RuvA subunit, N-terminal domain, E.coli), 1pfsA (Gene V protein, Pseudomonas bacteriophage pf3), 1gvp (Gene V protein, Filamentous bacteriophage (f1, M13)), 1gpc (Gene 32 protein (gp32) core, Bacteriophage T4), 1wgjA (Inorganic pyrophosphatase, S. cerevisiae), and 2prd (Inorganic pyrophosphatase, T. thermophilus).

Non-exhaustive examples of proteins with SH3 domaines are Signal transducing adaptor proteins, CDC24, Cdc25, PI3 kinase, Phospholipase, Ras GTPase-activating protein, Vav proto-oncogene, GRB2, p54 S6 kinase 2 (S6K2), SH3D21, C10orf76 (potentially), STAC3, Some myosins, SHANK1,2,3, ARHGAP12, C8orf46, TANGO1, Integrase, Focal Adhesion Kinase (FAK, PTK2), Proline-rich tyrosine kinase (Pyk2, CADTK, PTK2beta), or TRIP10 (cip4).

### Description of Leptin receptor (LepR)-binding variants based on Sac7d and Aho7c

Sac7d and Aho7c are proteins that have large similarity. One can also note that Sto7 (SEQ ID NO: 15) also present similarity with these proteins.

SEQ ID NO: 654 corresponds to the consensus sequence of after alignment of amino acids 2-66 of Sac7d (SEQ ID NO: 1) and 3-60 of Aho7c (SEQ ID NO: 14). The starting amino acids have been omitted as they are not essential in the structure of the proteins.

XKVKFKYKGEEKEVDXSKIKKVWRVGKMXSFTYDDNGKTGRGAVSEKDAPK
ELLXXXXXXXXXX (SEQ ID NO: 65)

In this consensus sequence, X (or Xaa) are as in Table 1.

**Table 1. Description of some amino acids represented as Xaa in SEQ ID NO: 59-73.**

| Position | Amino-acid | Preferred 1 Sac7d | Preferred 2 Aho7c |
|---|---|---|---|
| 1 | V or T | V | T |
| 16 | T or I | T | I |
| 29 | V or I | V | I |
| 55-64 | DMLARAEREK (SEQ ID NO: 78), | DMLARAEREK (SEQ ID NO: 78), | EKLK (SEQ ID NO: 80) |
| | DLLARAEREK | DLLARAEREK | |
| | (SEQ ID NO: 79) or EKLK------ (SEQ ID NO: 80) | (SEQ ID NO: 79) | |

Consensus sequence for variants binding to human leptin receptor are represented by

**Table 2: sequences of the variants based on the consensus sequence of Sac7d-Aho7c. Amino acids represented by X are further described in Tables 1, 3 and 4.**

| SEQ ID NO: | Sequence |
|---|---|
| 30 | XKVKFXXXGEEKEVXXSKIXXVYRXGKXXXFWYDDXGKXGXGH VXEKDAPKELLXXXXXXXXX |
| 31 | XKVKFXMXGEEKEVXXSKIXXVYRXGKXXXFWYDDXGKXGXG HVXEKDAPKELLXXXXXXXXXX |
| 32 | XKVKFXMGGEEKEVXXSKIXXVYRXGKXXGFWYDDXGKTGGG HVSEKDAPKELLXXXXXXXXXX |
| 33 | XKVKFRMGGEEKEVXXSKIRSVYRVGKFXGFWYDDXGKTGGG HVSEKDAPKELLXXXXXXXXXX |
| 34 | XKVKFVMGGEEKEVXXSKIDNVYRDGKHXGFWYDDXGKTGGG HVSEKDAPKELLXXXXXXXXXX |

**Table 3. description of amino acids indicated as Xaa in SEQ ID NO: 30-64. It is to be noted that this table is to be used for the sequences mentioned above, for the Xaa that they bear. For some sequences, an amino acid is specified at some of the positions of Table 3 and the line of the table is thus not applicable. The listing sequence depicts the Xaa of the second column of this table.**

| Position | Amino-acid | Preferred amino acid | Preferred 1 (F08) | Preferred 2 (D07) |
|---|---|---|---|---|
| 6 | Xaa is any amino acid | Xaa is any amino acid | R | V |
| 7 | Xaa is M, Q, T, V, I or A | M | M | M |
| 8 | Xaa is G, A or S | Xaa is G or A | G | G |
| 20 | Xaa is any amino acid | Xaa is any amino acid | R | D |
| 21 | Xaa is any amino acid | Xaa is D, N, S or A | S | N |
| 23 | Y | Y | Y | Y |
| 25 | Xaa is V, A, I, D or S | Xaa is V, A or D | V | D |
| 28 | Xaa is F, Y, H, M, V or A | Xaa is F, Y, H, or M | F | H |
| 30 | Xaa is G, A or S | Xaa is G or A | G | G |
| 32 | W | W | w | w |
| 39 | Xaa is T, Y, L or A | Xaa is T or Y | T | T |
| 41 | Xaa is G, A or S | Xaa is G or A | G | G |
| 43 | H | H | H | H |
| 45 | Xaa is S, H, K, Q or R | Xaa is S or K | S | S |

In these sequences, some amino acids that are not involved in binding can also be modified on the variants, without modifying the binding and biological properties of the proteins. They are represented in Table 4.

**Table 4. Amino acids represented as Xaa in SEQ ID NO: 30-64. Xaa at position 56 is only present in SEQ ID NO: 30-49.**

| Position | Amino acid |
|---|---|
| 15 | Xaa is D or E |
| 36 | Xaa is N or Q |
| 56 | Xaa is M or L |

### Description of leptin receptor-binding variants based on Sac7d (SEQ ID NO: 1)

Specific variants based on Sac7d, binding to the human leptin receptor are depicted by SEQ ID NO: 35 to SEQ ID NO: 49.

In these sequences, the starting methionine (M) of the Sac7d protein has been omitted. It has indeed been shown by the Applicant that the activity of the proteins is not modified when this amino acid is deleted. Likewise, it is possible to omit all or part of the last 7 amino acids from the variants and retain activity.

Consequently, proteins depicted by amino acids 1-57 of any of SEQ ID NO: 35 to SEQ ID NO: 49 are also variants according to the invention. One can also cite proteins depicted by amino acids 1-58, 1-59, 1-60, 1-61, 1-62, 1-63 of any of SEQ ID NO: 35 to SEQ ID NO: 49, which are also variants according to the invention.

Consequently, the polypeptide comprise any of SEQ ID NO: 35 to SEQ ID NO: 49, or any truncated protein based on these sequences, and as disclosed above.

Such variants are represented by

**Table 5: sequences of the variants based on Sac7d. Amino acids represented by X are further described in Tables 3 and 4.**

| SEQ ID NO: | Sequence |
|---|---|
| 35 | VKVKFXXXGEEKEVXTSKIXXVYRXGKXVXFWYDDXGKXGXGH VXEKDAPKELLDXLARAEREK |
| 36 | VKVKFXMXGEEKEVXTSKIXXVYRXGKXVXFWYDDXGKXGXG HVXEKDAPKELLDXLARAEREK |
| 37 | VKVKFXMGGEEKEVXTSKIXXVYRXGKXVGFWYDDXGKTGGG HVSEKDAPKELLDXLARAEREK |
| 38 | VKVKFRMGGEEKEVXTSKIRSVYRVGKFVGFWYDDXGKTGGG HVSEKDAPKELLDXLARAEREK |
| 39 | VKVKFVMGGEEKEVXTSKIDNVYRDGKHVGFWYDDXGKTGGG HVSEKDAPKELLDXLARAEREK |
| 40 | VKVKFXXXGEEKEVDTSKIXXVYRXGKXVXFWYDDNGKXGXGH VXEKDAPKELLDMLARAEREK |
| 41 | VKVKFXMXGEEKEVDTSKIXXVYRXGKXVXFWYDDNGKXGXG HVXEKDAPKELLDMLARAEREK |
| 42 | VKVKFXMGGEEKEVDTSKIXXVYRXGKXVGFWYDDNGKTGGG HVSEKDAPKELLDMLARAEREK |
| 43 | VKVKFRMGGEEKEVDTSKIRSVYRVGKFVGFWYDDNGKTGGG HVSEKDAPKELLDMLARAEREK |
| 44 | VKVKFVMGGEEKEVDTSKIDNVYRDGKHVGFWYDDNGKTGGG HVSEKDAPKELLDMLARAEREK |
| 45 | VKVKFXXXGEEKEVETSKIXXVYRXGKXVXFWYDDQGKXGXGH VXEKDAPKELLDLLARAEREK |
| 46 | VKVKFXMXGEEKEVETSKIXXVYRXGKXVXFWYDDQGKXGXG |
| | HVXEKDAPKELLDLLARAEREK |
| 47 | VKVKFXMGGEEKEVETSKIXXVYRXGKXVGFWYDDQGKTGGG HVSEKDAPKELLDLLARAEREK |
| 48 | VKVKFRMGGEEKEVETSKIRSVYRVGKFVGFWYDDQGKTGGG HVSEKDAPKELLDLLARAEREK |
| 49 | VKVKFVMGGEEKEVETSKIDNVYRDGKHVGFWYDDQGKTGGG HVSEKDAPKELLDLLARAEREK |

As indicated above, it is possible to modify D16 of Sac7d (which is located at position 15 in SEQ ID NO: 35 to SEQ ID NO: 49, as the M1 present in Sac7d has been omitted), N37 of Sac7d (herein located at position 36) or M57 of Sac7d (herein located at position 56) as disclosed in Table 4.

As for the consensus sequence of Sac7d / Aho7c, any combination of substitution is foreseen (numbering is with respect to SEQ ID NO: 35 to SEQ ID NO: 49), even though the sequence listing doesn't all depict them:

| | | | |
|---|---|---|---|
| D15 | N36 | M56 | SEQ ID NO: 40-44 |
| D15 | N36 | L56 | |
| D15 | Q36 | M56 | |
| D15 | Q36 | L56 | |
| E15 | N36 | M56 | |
| E15 | N36 | L56 | |
| E15 | Q36 | M56 | |
| E15 | Q36 | L56 | SEQ ID NO: 45-49 |

As indicated above, the sequences all contain the mutated amino acids Y23, W32 and H43 (corresponding respectively to position 24, 33 and 44 of SEQ ID NO: 1, which contain the N-terminus methionine), which differ from the amino acids that are naturally present in Sac7d.

The Applicant has indeed found that these amino acids are present in various variants binding to human leptin receptor, and that modification of such leads to decrease of binding (loss of affinity or loss of binding). The other amino acids can be variable, under conditions mentioned in Table 3.

It is preferred when the variants contain the M7 (corresponding to position 8 of SEQ ID NO: 1), T39 (corresponding to position 40 of SEQ ID NO: 1) and/or S45 (corresponding to position 46 of SEQ ID NO: 1).

Very interesting variants are depicted by SEQ ID NO: 38, SEQ ID NO: 39, SEQ ID NO: 43, SEQ ID NO: 44, SEQ ID NO: 48 and SEQ ID NO: 49.

### Description of proteins based on Aho7c (SEQ ID NO: 14)

Variants of Aho7c, binding to human leptin receptor are depicted by SEQ ID NO: 50 to SEQ ID NO: 64. As indicated above, Aho7c is very similar to Sac7d. It has also been shown and reminded herein that mutations of Sac7d can be carried from Sac7d to another protein (WO 2012/150314).

In these sequences, the starting methionine and alanine (MA) of the Aho7c protein (SEQ ID NO: 14) have been omitted. It has indeed been shown by the Applicant that the activity of the proteins is not modified when these amino acids are deleted. Likewise, it is possible to omit all or part of the last 4 amino acids from the variants and retain activity.

Consequently, proteins depicted by amino acids 1-55 of any of SEQ ID NO: 50 to SEQ ID NO: 64 are also variants according to the invention. One can also cite proteins depicted by amino acids 1-56, 1-57, 1-58 of any of SEQ ID NO: 50 to SEQ ID NO: 64, which are also variants according to the invention.

Consequently, the polypeptide comprise any of SEQ ID NO: 50 to SEQ ID NO: 64, or any truncated protein based on these sequences, and as disclosed above.

Such variants are represented by

**Table 6: sequences of the variants based on Aho7c. Amino acids represented by X are further described in Tables 3 and 4.**

| SEQ ID NO: | Sequence |
|---|---|
| 50 | TKVKFXXXGEEKEVXISKIXXVYRXGKXIXFWYDDXGKXGXGHV XEKDAPKELLEKLK |
| 51 | TKVKFXMXGEEKEVXISKIXXVYRXGKXIXFWYDDXGKXGXGHV XEKDAPKELLEKLK |
| 52 | TKVKFXMGGEEKEVXISKIXXVYRXGKXIGFWYDDXGKTGGGH VSEKDAPKELLEKLK |
| 53 | TKVKFRMGGEEKEVXISKIRSVYRVGKFIGFWYDDXGKTGGGH VSEKDAPKELLEKLK |
| 54 | TKVKFVMGGEEKEVXISKIDNVYRDGKHIGFWYDDXGKTGGGH VSEKDAPKELLEKLK |
| 55 | TKVKFXXXGEEKEVDISKIXXVYRXGKXIXFWYDDNGKXGXGHV XEKDAPKELLEKLK |
| 56 | TKVKFXMXGEEKEVDISKIXXVYRXGKXIXFWYDDNGKXGXGH VXEKDAPKELLEKLK |
| 57 | TKVKFXMGGEEKEVDISKIXXVYRXGKXIGFWYDDNGKTGGGH VSEKDAPKELLEKLK |
| 58 | TKVKFRMGGEEKEVDISKIRSVYRVGKFIGFWYDDNGKTGGGH VSEKDAPKELLEKLK |
| 59 | TKVKFVMGGEEKEVDISKIDNVYRDGKHIGFWYDDNGKTGGGH VSEKDAPKELLEKLK |
| 60 | TKVKFXXXGEEKEVEISKIXXVYRXGKXIXFWYDDQGKXGXGHV XEKDAPKELLEKLK |
| 61 | TKVKFXMXGEEKEVEISKIXXVYRXGKXIXFWYDDQGKXGXGH VXEKDAPKELLEKLK |
| 62 | TKVKFXMGGEEKEVEISKIXXVYRXGKXIGFWYDDQGKTGGGH VSEKDAPKELLEKLK |
| 63 | TKVKFRMGGEEKEVEISKIRSVYRVGKFIGFWYDDQGKTGGGH VSEKDAPKELLEKLK |
| 64 | TKVKFVMGGEEKEVEISKIDNVYRDGKHIGFWYDDQGKTGGGH VSEKDAPKELLEKLK |

As indicated above, it is possible to modify D17 of Aho7c (which is located at position 15 in SEQ ID NO: 50 to SEQ ID NO: 64, as the M1A2 present in Aho7c have been omitted), or N38 of Aho7c (herein located at position 36) as disclosed in Table 4.

As for the consensus sequence of Sac7d / Aho7c, any combination of substitution is foreseen (numbering is with respect to SEQ ID NO: 50 to SEQ ID NO: 64), even though the sequence listing doesn't all depict them:

| | | |
|---|---|---|
| D15 | N36 | SEQ ID NO: 55-59 |
| D15 | Q36 | |
| E15 | N36 | |
| E15 | Q36 | SEQ ID NO: 60-64 |

As indicated above, the sequences all contain the mutated amino acids Y23, W32 and H43 (corresponding respectively to position 25, 34 and 45 of SEQ ID NO: 14, which contain the N-terminus methionine and alanine), which differ from the amino acids that are naturally present in Sac7d.

The Applicant has indeed found that these amino acids are present in various variants binding to human leptin receptor, and that modification of such leads to decrease of binding (loss of affinity or loss of binding). The other amino acids can be variable, under conditions mentioned in Table 3.

It is preferred when the variants contain the M7 (corresponding to position 9 of SEQ ID NO: 14), T39 (corresponding to position 41 of SEQ ID NO: 14) and/or S45 (corresponding to position 47 of SEQ ID NO: 14).

Very interesting variants are depicted by SEQ ID NO: 53, SEQ ID NO: 54, SEQ ID NO: 58, SEQ ID NO: 59, SEQ ID NO: 63 and SEQ ID NO: 64.

### Description of proteins based on Sso7d (SEQ ID NO: 2)

Variants based on Sso7d, binding to human leptin receptor are depicted by SEQ ID NO: 66 to SEQ ID NO: 70. As indicated above, Sso7d is very similar to Sac7d. It has also been shown that mutations of Sac7d can be carried to Sso7d (WO 2012/150314).

In these sequences, the starting methionine, alanine and threonine (MAT) of the Sso7d protein (SEQ ID NO: 2) have been omitted. It has indeed been shown by the Applicant that the activity of the proteins is not modified when these amino acids are deleted. Likewise, it is possible to omit all or part of the last 6 amino acids from the variants and retain activity.

Consequently, proteins depicted by amino acids 1-53 of any of SEQ ID NO: 66 to SEQ ID NO: 70 are also variants according to the invention. One can also cite proteins depicted by amino acids 1-54, 1-55, 1-56, 1-57 or 1-58 of any of SEQ ID NO: 66 to SEQ ID NO: 70, which are also variants according to the invention.

Consequently, the polypeptide comprise any of SEQ ID NO: 66 to SEQ ID NO: 70, or any truncated protein based on these sequences, and as disclosed above.

Such variants are represented by

**Table 7: sequences of the variants based on Sso7d. Amino acids represented by X are further described in Table 8.**

| SEQ ID NO: | Sequence |
|---|---|
| 66 | VKFXXXGEEKEVDISKIXXVYRXGKXIXFWYDEGGGKXGXHV XEKDAPKELLQLLEKQ |
| 67 | VKFXMXGEEKEVDISKIXXVYRXGKXIXFWYDEGGGKXGXGHV XEKDAPKELLQLLEKQ |
| 68 | VKFXMGGEEKEVDISKIXXVYRXGKXIGFWYDEGGGKTGGGHV SEKDAPKELLQLLEKQ |
| 69 | VKFRMGGEEKEVDISKIRSVYRVGKFIGFWYDEGGGKTGGGHV SEKDAPKELLQLLEKQ |
| 70 | VKFVMGGEEKEVDISKIDNVYRDGKHIGFWYDEGGGKTGGGH VSEKDAPKELLQLLEKQ |

**Table 8. Description of amino acids indicated as Xaa in SEQ ID NO: 66-70. It is to be noted that this table is to be used for the sequences mentioned above, for the Xaa that they bear. For some sequences, an amino acid is specified at some of the positions of Table 8 and the line of the table is thus not applicable. The listing sequence depicts the Xaa of the second column of this table.**

| Position | Amino-acid | Preferred amino acid | Preferred 1 (F08) | Preferred 2 (D07) |
|---|---|---|---|---|
| 4 | Xaa is any amino acid | Xaa is any amino acid | R | V |
| 5 | Xaa is M, Q, T, V, I or A | M | M | M |
| 6 | Xaa is G, A or S | Xaa is G or A | G | G |
| 18 | Xaa is any amino acid | Xaa is any amino acid | R | D |
| 19 | Xaa is any amino acid | Xaa is D, N, S or A | S | N |
| 21 | Y | Y | Y | Y |
| 23 | Xaa is V, A, I, D or S | Xaa is V, A or D | V | D |
| 26 | Xaa is F, Y, H, M, V or A | Xaa is F, Y, H, or M | F | H |
| 28 | Xaa is G, A or S | Xaa is G or A | G | G |
| 30 | W | W | W | W |
| 38 | Xaa is T, Y, L or A | Xaa is T or Y | T | T |
| 40 | Xaa is G, A or S | Xaa is G or A | G | G |
| 42 | H | H | H | H |
| 44 | Xaa is S, H, K, Q or R | Xaa is S or K | S | S |

As indicated above, it is possible to modify D18 of Sso7d (which is located at position 13 in SEQ ID NO: 66 to SEQ ID NO: 68, as the M1A2T3 present in Sso7d have been omitted).

As indicated above, the sequences all contain the mutated amino acids Y21, W30 and H42 (corresponding respectively to position 24, 33 and 45 of SEQ ID NO: 14, which contain the N-terminus methionine, alanine and threonine), which differ from the amino acids that are naturally present in Sso7d.

The Applicant has indeed found that these amino acids are present in various variants binding to human leptin receptor, and that modification of such leads to decrease of binding (loss of affinity or loss of binding). The other amino acids can be variable, under conditions mentioned in Table 3.

It is preferred when the variants contain the M5 (corresponding to position 8 of SEQ ID NO: 2), T38 (corresponding to position 41 of SEQ ID NO: 2) and/or S44 (corresponding to position 47 of SEQ ID NO: 2).

Very interesting variants are depicted by SEQ ID NO: 69 and SEQ ID NO: 70.

### Description of variants based on the consensus sequence of the Sac7d family (SEQ ID NO: 16)

Variants for specific members of the Sac7d family are disclosed above. In view of the similarity of the sequences of Sac7d family proteins, as shown in Figure 1 and of the fact that it is possible to carry the mutations from one protein to another, the mutations can be depicted in the consensus sequence for the Sac7d family.

In sequences SEQ ID NO: 17-SEQ ID NO: 29, the amino acids designated as X or Xaa at positions 8-10, 22, 23, 27, 30, 32, 42, 44 and 48 are as indicated in Table 9. The other amino acids designated as X (or Xaa) are indicated in Table 10 or Table 11.

**Table 9. description of amino acids in SEQ ID NO: 17-SEQ ID NO: 29**

| **Position** | **Amino acid** |
|---|---|
| 2 | X is V, A or T |
| 3 | X is T or K |
| 4 | X is - or K |
| 6 | X is R or K |
| 15 | X is E or Q |
| 18 | X is T or I |
| 31 | X is V or I |
| 37 to 39 | XXX is EGG or DN- |
| 57 | X is M or L |
| 58 | X is Q, D or E |
| 59 | X is M or K |
| 61 to 67 | -------K, EKS-GKK (SEQ ID NO: 71), EK---QKK (SEQ ID NO: 72), ARAEREKK (SEQ ID NO: 73), ARAERE-K (SEQ ID NO: 74), ARAERE-- (SEQ ID NO: 75), ARA-EKKK (SEQ ID NO: 76), E-----KK, EKS-GKK (SEQ ID NO: 77) |

The amino acids disclosed in Table 1 correspond to the variability observed for proteins of the Sac7d family, as also shown in the consensus sequence of Figure 1.

The amino acids disclosed in Table 10 correspond to the amino acids that are involved in binding to human leptin receptor, in the variants herein disclosed. It is to be noted that such variants all differ from the wild-type proteins for the following amino acids: Position 25: presence of Y; position 34: presence of W; position 46: presence of H.

These amino acids have been shown by the inventors as important for the binding to human leptin receptor (change of the amino acid drastically decreases the ability to bind to albumin or the affinity to the albumin). In contrast, it is possible to modify the other amino acids from the optimized binders (SEQ ID NO: 20, SEQ ID NO: 21, SEQ ID NO: 24, SEQ ID NO: 25, SEQ ID NO: 28 or SEQ ID NO: 29), according to the teachings of Table 10. In particular it has been noted that replacement of the amino acids at positions 8, 22 or 23 of these binders by another amino acids essentially didn't alter binding or its characteristics. According to the inventor's analysis, amino acids at position 9 seems also to be tolerant to any amino acids. Amino acids at positions 10, 32 are preferably tiny, and amino acid at position 10 is preferably small. Amino acids at position 30 and 42 are preferably hydrophobic, and amino acid at position 48 is preferably polar. All information above can easily be translated to specific members of the Sac7d family disclosed above and corresponding amino acids in tables 3 and 8.

It has also been shown that the D17 (aspartic acid) of the consensus sequence (and which is not involved in binding) can be replaced by a E (glutamic acid). This is represented in the sequences mentioned above (SEQ ID NO: 17-SEQ ID NO: 21 bearing an X in position 17; SEQ ID NO: 22-SEQ ID NO: 25 bearing a D17; SEQ ID NO: 26-SEQ ID NO: 29 bearing a E17).

**Table 10. description of amino acids indicated as Xaa in SEQ ID NO: 17-SEQ ID NO: 29. It is to be noted that this table is to be used for the sequences mentioned above, for the Xaa that they bear. For some sequences, an amino acid is specified at some of the positions of Table 10 and the line of the table is thus not applicable.**

| Position | Amino-acid | Preferred amino acid | Preferred 1 (F08) | Preferred 2 (D07) |
|---|---|---|---|---|
| 8 | Xaa is any amino acid | Xaa is any amino acid | R | V |
| 9 | Xaa is M, Q, T, V, I or A | M | M | M |
| 10 | Xaa is G, A or S | Xaa is G or A | G | G |
| 22 | Xaa is any amino acid | Xaa is any amino acid | R | D |
| 23 | Xaa is any amino acid | Xaa is D, N, S or A | S | N |
| 25 | Y | Y | Y | Y |
| 27 | Xaa is V, A, I, D or S | Xaa is V, A or D | V | D |
| 30 | Xaa is F, Y, H, M, V or A | Xaa is F, Y, H, or M | F | H |
| 32 | Xaa is G, A or S | Xaa is G or A | G | G |
| 34 | W | W | W | W |
| 42 | Xaa is T, Y, L or A | Xaa is T or Y | T | T |
| 44 | Xaa is G, A or S | Xaa is G or A | G | G |
| 46 | H | H | H | H |
| 48 | Xaa is S, H, K, Q or R | Xaa is S or K | S | S |

As indicated above, the methionine at position 1 of SEQ ID NO: 17-29 can be omitted. So can amino acids 61-68 of SEQ ID NO: 17-29.

In one embodiment, the polypeptide comprises SEQ ID NO: 17 or amino acids 2-60 of one of the sequence from Table 11.

**Table 11. List of variants sequences based on the consensus sequence of the proteins of the Sac7d family. The nature of X is provided in Tables 9 and 10. X at position 17 can be E or D.**

| SEQ ID NO: | Sequence |
|---|---|
| 17 | MXXXVXFXXXGEEKXVXXSKIXXVYRXGKXXXFWYDXXXGKXG XGHVXEKDAPKELXXXLXXXXXXXX |
| 18 | MXXXVXFXMXGEEKXVXXSKIXXVYRXGKXXXFWYDXXXGKX GXGHVXEKDAPKELXXXLXXXXXXXX |
| 19 | MXXXVXFXMGGEEKXVXXSKIXXVYRXGKXXGFWYDXXXGKT GGGHVSEKDAPKELXXXLXXXXXXXX |
| 20 | MXXXVXFRMGGEEKXVXXSKIRSVYRVGKFXGFWYDXXXGKT GGGHVSEKDAPKELXXXLXXXXXXXX |
| 21 | MXXXVXFVMGGEEKXVXXSKIDNVYRDGKHXGFWYDXXXGKT GGGHVSEKDAPKELXXXLXXXXXXXX |
| 22 | MXXXVXFXXXGEEKXVDXSKIXXVYRXGKXXXFWYDXXXGKXG XGHVXEKDAPKELXXXLXXXXXXX |
| 23 | MXXXVXFXMGGEEKXVDXSKIXXVYRXGKXXGFWYDXXXGKT GGGHVSEKDAPKELXXXLXXXXXXXX |
| 24 | MXXXVXFRMGGEEKXVDXSKIRSVYRVGKFXGFWYDXXXGKT GGGHVSEKDAPKELXXXLXXXXXXXX |
| 25 | MXXXVXFVMGGEEKXVDXSKIDNVYRDGKHXGFWYDXXXGKT |
| | GGGHVSEKDAPKELXXXLXXXXXXXX |
| 26 | MXXXVXFXXXGEEKXVEXSKIXXVYRXGKXXXFWYDXXXGKXG XGHVXEKDAPKELXXXLXXXXXXXX |
| 27 | MXXXVXFXMGGEEKXVEXSKIXXVYRXGKXXGFWYDXXXGKT GGGHVSEKDAPKELXXXLXXXXXXXX |
| 28 | MXXXVXFRMGGEEKXVEXSKIRSVYRVGKFXGFWYDXXXGKT GGGHVSEKDAPKELXXXLXXXXXXXX |
| 29 | MXXXVXFVMGGEEKXVEXSKIDNVYRDGKHXGFWYDXXXGKT GGGHVSEKDAPKELXXXLXXXXXXXX |

### Production of the identified variants

The sequence of the identified variant can be cloned in any appropriate vector by any molecular genetic methods known in the art.

These recombinant DNA constructs comprising a nucleotide sequence, coding for a polypeptide comprising a variant as described above, are used in connection with a vector, such as a plasmid, phagemid, phage or viral vector.

These recombinant acid molecules can be produced by techniques described in Sambrook et al., 1989 (Sambrook J, Fritsch EF and Maniatis T (1989) Molecular cloning: a laboratory manual, Cold Spring Harbor Laboratory Press, New York). Alternatively, the DNA sequences may be chemically synthesized using, for example, synthesizers.

Recombinant constructs of the invention comprise the expression vectors that are capable of expressing the RNA and thus lead to production of proteins from the above genetic sequences. The vector may thus further comprise regulatory sequences, including a suitable promoter operably linked to the open reading frame (ORF) of the genetic sequences herein disclosed. The vector may further comprise a selectable marker sequence such as an antibiotic resistance gene. Specific initiation and bacterial secretory signals also may be required for efficient translation of the coding sequences when bacteria as used as the expression host.

### Production of the molecules

Cells are transfected or transformed with vectors containing the sequences coding for the polypeptides comprising the variant as disclosed above.

The cells are the cultured in such conditions as to have the protein expressed and favorably secreted. The conditions of culture of the cells are the conditions generally used for recombinant antibody production and are known in the art. Such conditions that are known in the art can also be optimized by the person skilled in the art if needed. Kunert and Reinhart (Appl Microbiol Biotechnol. 2016; 100: 3451-3461) review such methods and provide ample references thereto.

One can use bacterial, phage (Shukra et al, Eur J Microbiol Immunol (Bp). 2014; 4(2): 91-98) or eukaryotic systems of production.

One shall prefer to use eukaryotic cells in order to obtain proper post-translational modifications such as glycosylation.

In particular, one can use CHO (Chinese Hamster Ovary) cells, PER.C6 cells (human cell line, Pau et al, Vaccine. 2001 21;19(17-19):2716-21), HEK 293b cells (Human embryonic kidney cells 293), NS0 cells (cell line derived from the non-secreting murine myeloma) or EB66 cells (a duck cell line Valneva, Lyons, France).

Also provided by the present disclosure are host cells containing at least one of the DNAs constructs coding for a polypeptide comprising the variant as disclosed herein. The host cell can be any cell for which expression vectors are available. As indicated above, it may be a higher eukaryotic host cell, such as a mammalian cell, a lower eukaryotic host cell, such as a yeast cell, or a prokaryotic cell, such as a bacterial cell.

Introduction of the recombinant construct into the host cell is performed by any method known in the art (such as calcium phosphate transfection, lipofection, DEAE, dextran mediated transfection, electroporation or phage infection). The vectors can be inserted within the genome of the host cell, or be maintained as an extragenomic vector (such as a Bacterial Artificial Chromosome or a Yeast Artificial Chromosome). When introduced within the cell genome, such introduction may be random or targeted using methods known in the art (homologous recombination or the like).

### Bacterial hosts and expression

Useful expression vectors for bacterial use are constructed by inserting the recombinant DNA sequence together with suitable translation initiation and termination signals in operable reading phase with a functional promoter. The vector will comprise one or more phenotypic selectable markers and an origin of replication to ensure maintenance of the vector and, if desirable, to provide amplification within the host.

Suitable prokaryotic hosts for transformation include *E. coli, Bacillus subtilis, Salmonella typhimurium* and various species within the genera *Pseudomonas, Streptomyces,* and *Staphylococcus.*

### Eukaryotic hosts and expression

Examples of the eukaryotic host cells include vertebrate cells, insect cells, and yeast cells. In particular, one can use the cells mentioned above.

The transformed or transfected cells are cultured according to methods known in the art and the polypeptide are recovered from intracellular or extracellular fractions (depending on whether it is secreted or not).

### Isolation of the molecules

The recombinant protein produced can be separated and purified by any of various known separation methods utilizing the physical or chemical property of the protein, from the intracellular or extracellular fraction.

In particular, one can use methods such as precipitation, ultrafiltration, various types of liquid chromatography such as molecular sieve chromatography (gel filtration), adsorption chromatography, ion exchange chromatography, and affinity chromatography, dialysis, and a combination thereof.

In general, any method known and used to purify recombinant polypeptides is adapted for the purification of the molecules herein disclosed.

If a tag has been introduced within the recombinant sequence (such as a poly-Histidine tag), one can purify the molecules using this tag. However, in certain embodiments, it is preferred to use affinity to purify the molecules.

One can, in particular, use the fact that the molecule herein produced binds to a specific target and use any affinity method (affinity column, FACS, beads) to isolate such molecules.

One particular advantage of the molecules herein disclosed is that they don't need to be glycosylated to be active and can thus be produced in any type of cells, and not necessarily eukaryotic cells. They are particularly well produced in bacterial cells.

### Modification of the variants

The variants described above, and having the ability to bind human leptin receptor can be modified by any method known in the art.

### Preparing a polypeptide comprising the variant

It is possible to prepare a DNA sequence containing two coding sequences, in frame, one for the variant herein disclosed and another for a protein or peptide of interest. The resulting expressed protein would thus be a polypeptide that contains both proteins. The vector may be built in such a way that it would contain a sequence coding for a linker that would be located between the two proteins in the expressed polypeptide.

Consequently, the invention also encompasses a polypeptide comprising the variant of a protein of the OB-fold protein (preferably of the Sac7d family) binding to human leptin receptor which is fused or linked (preferably through amine bond as disclosed above) to another protein or polypeptide.

In a specific embodiment, the other protein or polypeptide comprises another variant of a protein of the Sac7d family, in particular as herein disclosed. In other embodiments, the other protein or polypeptide comprises at least two other variants of a protein of the Sac7d family. In some embodiment, the variant of a protein of the Sac7d family binding to human leptin receptor is bound to another variant of the Sac7d family at its N- and C-terminus. The variant may bind to a target as described below for antibodies.

Are of particular interest
- A polypeptide comprising a variant of an OB-fold protein of the Sac7d family binding to human leptin receptor, fused with a variant of an OB-fold protein of the Sac7d family binding to albumin and to a variant of an OB-fold protein of the Sac7d family binding to PD-L1 (in particular the ones disclosed in WO2021180823). Such polypeptides are particularly useful for the treatment of cancers, in particular of non-small cell lung cancer, metastatic merkel-cell carcinoma, urothelial carcinoma, solid tumors, hematologic cancers and in particular lymphomas (notably classical Hodgkin lymphoma), cutaneous squamous cell carcinoma, squamous cell lung cancer, renal cell carcinoma or melanoma.
- A polypeptide comprising a variant of an OB-fold protein of the Sac7d family binding to human leptin receptor, fused with a variant of an OB-fold protein of the Sac7d family binding to albumin and to a variant of an OB-fold protein of the Sac7d family binding to Tumor Necrosis Factor alpha - TNF-alpha) or RANK-L (in particular the ones disclosed in WO2020074402). Such polypeptides are particularly useful for the treatment of immunologic or inflammation diseases (such as rheumatoid arthritis, ankylosing spondylitis, psoriasis and psoriatic arthritis, Crohn disease, ulcerative colitis, coeliac disease, glomerulonephritis, hepatitis, inflammatory bowel disease), central nervous system (in particular demyelinating diseases such as multiple sclerosis, optic neuritis or nondemyelinating diseases such as meningitis, meningoencephalitis, encephalitis, neurosarcoidosis, or CNS vasculitis), respiratory diseases (such as idiopathic pulmonary fibrosis, asthma, chronic obstructive pulmonary disease (COPD), pulmonary Langerhans' cell histiocytosis, emphysema, and pulmonary fibrosis), osteoporosis or treatment of covid-19.
- A polypeptide comprising a variant of an OB-fold protein of the Sac7d family binding to human leptin receptor, fused with a variant of an OB-fold protein of the Sac7d family binding to albumin and to a variant of an OB-fold protein of the Sac7d family binding to IL-17 (in particular the ones disclosed in WO2019096797) for the treatment of the same diseases as mentioned for TNF-alpha

In another embodiment, the other protein or polypeptide is an antibody. In this embodiment, the variant of the OB-fold domain is fused to at least one of the heavy or light chain of an immunoglobulin monomer, preferably at the N- or C-terminus of the light or heavy chain. In another embodiment, the variant may be fused to both heavy or light chains.

In order to obtain such compounds, one can use a genetic construct comprising a DNA sequence selected from the group consisting of
a. the sequence coding for the heavy chain of an antibody fused, at its 3'end with the sequence coding for the variant of an OB-fold protein (potentially with an sequence coding for a linker)
b. the sequence coding for the heavy chain of an antibody fused, at its 5' end with the sequence coding for the variant of an OB-fold protein (potentially with an sequence coding for a linker)
c. the sequence coding for the light chain of an antibody fused, at its 3' end with the sequence coding for the variant of an OB-fold protein (potentially with an sequence coding for a linker)
d. the sequence coding for the light chain of an antibody fused, at its 5' end with the sequence coding for the variant of an OB-fold protein (potentially with an sequence coding for a linker).

This fusion can be made at the N-terminus and/or the C-terminus of the antibody chain (heavy and/or light chain). It is to be noted that, especially when using a small OB-fold domain (about 70 amino acids) such as a protein from the Sac7d family, it is possible to obtain a molecule that will have the structure of the antibody (two light chains paired to two heavy chains, and such dimers paired together), with the antibody regions, and further binding regions consisting of the modified OB-fold domain.

In a specific embodiment, the antibody part of the protein herein disclosed is a IgG molecule.

In another embodiment, the antibody part of the protein herein disclosed is a IgA molecule.

In another embodiment, the antibody part of the protein herein disclosed is a IgM molecule.

In another embodiment, the antibody part of the protein herein disclosed is a IgD molecule.

In another embodiment, the antibody part of the protein herein disclosed is a IgE molecule.

The antibody may be a human antibody, a rodent antibody (such as a mouse antibody or a rat antibody), a cat antibody, a dog antibody, a chicken antibody, a goat antibody, a camelid antibody (such as a camel antibody, a llama antibody, an alpaca antibody or a nanobody), a shark antibody, or an antibody from any other species. It may be a chimeric or humanized antibody. As reminded in Wikipedia, humanized antibodies are antibodies from non-human species whose protein sequences have been modified to increase their similarity to antibody variants produced naturally in humans. A chimeric antibody contains sequences from different species.

It is preferred when the antibody that is part of the molecule herein disclosed is an antibody that comprises two identical heavy chains (of about 400 to 500 amino acids, generally around 450 amino acids) and two identical light chains. Consequently, the antibody comprises the same Fab variable regions. This antibody is therefore a monospecific antibody where both parts (combination of light and heavy chains) of the antibody bind to the same epitope of an antigen.

However, the antibody may present different heavy and/or light chains. In particular, in some embodiments, the antibody is a bi-specific antibody. The term "antibody" thus encompasses both "classical antibodies" as disclosed above having identical heavy and light chains, but also engineered antibodies that have more than one specificity.

In a specific embodiment, the antibody presents one heavy and light chain from one antibody and another heavy and light chain from another antibody.

The antibody may bind to a target selected in the group consisting of:
Cell surface receptors: Insulin receptor, Low density lipoprotein receptor-related protein 1, Transferrin receptor, Epidermal growth factor receptor, Epidermal growth factor receptor variant III, Vascular endothelial growth factor receptor 1, Vascular endothelial growth factor receptor 2, Her2, Her3, Her4, PSMA, IGF-1R, GITR, RAGE, CD28, FcRn, Platelet-derived growth factor receptor.
Cell surface proteins: CD19, CD20, CD22, CD30, CD38, CD40, CD248, CD47, CD73, CD3, TIM-3, CEA, cMet, ICAM1, ICAM3, MadCam, a4b7, CD7, CD4, CD223, CD138.
Angiogenesis factors and Growth factors: VEGF, Angiopoietin 2, HGF, PDGF, EGF, GM-CSF, HB-EGF, TGF
Immune checkpoint inhibitors or activators: PD-1, PD-L1, CTLA4, CD28, B7-1, B7-2, ICOS, ICOSL, B7-H3, B7-H4, LAG3, KIR, 4-1BB, OX40, CD27, CD40L, TIM3, A2aR, complement component 5
Circulating proteins: HSA, TNFa, IL23, IL12, IL33, IL4, IL13, IL5, IL6, IL4, IL1, IL22, IL36, IFNg, IL17, RANKL, Bace1, alpha Synuclein, Tau, amyloid, coagulation factor XI, coagulation factor XII, IgE, LPAM.

In another embodiment, the variant of the OB-fold domain (in particular the variant of a protein of the Sac7d family) is conjugated to an organic molecule. This may be done by any method known in the art. In particular, one can chemically link the molecule to the protein. As a molecule, one can cite antiproliferative (cytotoxic and cytostatic agents) include cytotoxic compounds (e.g., broad spectrum), angiogenesis inhibitors cell cycle progression inhibitors, PBK/m-TOR/AKT pathway inhibitors, MAPK signaling pathway inhibitors, kinase inhibitors, protein chaperones inhibitors, HDAC inhibitors, PARP inhibitors, Wnt/Hedgehog signaling pathway inhibitors, RNA polymerase inhibitors and proteasome inhibitors. One can also use anti-inflammatory molecules.

One can cite in particular DNA-binding or alkylating drugs such as anthracyclines (doxorubicin, epirubicin, idarubicin, daunorubicin) and its analogs, alkylating agents, such as calicheamicins, dactinomycines, mitromycines, pyrrolobenzodiazepines, and the like. One can also cite cell cycle progression inhibitors such as CDK inhibitors, Rho-kinase inhibitors, checkpoint kinase inhibitors, aurora kinase inhibitors, PLK inhibitors, and KSP inhibitors. One can also cite thalidomide and its derivatives lenalidomide and pomalidomide. In order for treating inflammation disorders, one can also use cyclooxygenase-2 inhibitors, 5-lipoxygenase inhibitors, quercetin and/or resveratrol as molecules conjugated to the polypeptide comprising the variant.

Interesting and preferred molecules are also disclosed above.

### Use of the variants

The variants can be used in particular in therapeutic methods, when fused to another polypeptide that has therapeutic activity for treating a given disease.

Biologicals have been developed for the treatment of multiple diseases and can be fused to the LepR-binding variants herein disclosed, for the treatment of cancer, auto-immune diseases, inflammatory diseases or infectious diseases. One can cite, as illustration, trastuzumab for the treatment of Her2/neu breast cancer. Various other therapeutic antibodies are listed in WO2019096797 (pages 16-18, herein incorporated as reference) and can also be used with the LepR-binding variants herein disclosed.

The invention thus relates to a method of treatment of a specific disease, comprising the administration of a therapeutic amount of a LepR-binding variant of an OB-fold herein disclosed (in particular a variant of a protein of the Sac7d family), fused to a therapeutic agent active against the specific disease, to a subject in need thereof.

The term "therapeutic amount" or "effective amount", as used herein, is the amount sufficient to effect beneficial or desired results, such as clinical results, and an "effective amount" depends upon the context in which it is being applied. An effective amount is an amount that provides therapeutic improvement while minimizing side or adverse effect. Therapeutic improvement may be regression of the disease, improvement in life quality of the subject, improvement of the efficacy of a combined treatment.

In the context of administering a polypeptide comprising the variant binding to leptin receptor and a peptide or polypeptide having a therapeutic activity, to a patient with a disease targeted by the peptide or polypeptide having a therapeutic activity, an effective amount of is, for example, an amount sufficient to achieve cure of the patient, improvement of the patient's condition, acceleration of the patient's cure, as compared to the response obtained without administration. It can also be an amount that stops or slows the progression of the disease. It can be an amount that allows regression of the disease, or of biological markers associated with the specific disease. It is obtained by allowing passage through the intestinal barrier of a quantity of the variant fused to the peptide or polypeptide having a therapeutic activity so that the concentration/amount of the peptide or polypeptide having a therapeutic activity at its site of action is sufficient to provide a therapeutic effect.

An agent with therapeutic activity (or a therapeutic agent) is an agent which, upon administration at an efficient amount to a subject having a disease improves the condition of the subject, or decreases a biomarker linked to the disease.

One can administer the variant by any method of the art.

However, it is preferred when the variant is administered by the oral route as disclosed in WO 2016/062874. In this embodiment, the variant, and any other element bound thereto is formulated with pharmaceutically acceptable excipients for oral administration. Such formulations are known in the art.

In another embodiment, the variant is administered by the rectal route. In this embodiment, the variant, and any other element bound thereto is formulated with pharmaceutically acceptable excipients for rectal administration. Such formulations are known in the art.

In some embodiments, a polypeptide or variant (or nucleotide encoding a polypeptide or variant) may be associated (e.g., physically associated) with a delivery agent such as a nanoparticle (e.g., lipid nanoparticle), a dendrimer, a polymer, a liposome, or a cationic delivery system. It is particularly interesting when the other polypeptide or organic molecule bound to the LepR-binding variant is protected from gastric degradation through an appropriate coating or by encapsulation in an appropriate vector. In this case, the LepR-binding variant is bound to this protecting element. Indeed, WO2016062874 shows that the variants of the Sac7d family are resistant to gastric degradation (enzymatic or pH degradation). The variant thus doesn't need to be protected. However, in some embodiments, both the LepR-binding variant bound to the therapeutically active molecule are encapsulated/protected from gastric degradation. In this case, the protection is to allow release of the LepR-binding variant bound to the therapeutically active molecule in the intestine.

The variants or polypeptides containing the variants can also be used in diagnostic methods. In particular, such variants or polypeptide may be linked to any marker known in the art and used in imagery methods.

The invention thus also relates to a method for detecting the presence of, or for quantifying, human leptin receptor in a sample, comprising the step of
a. Exposing the sample to a variant as disclosed that binds to human leptin receptor, in such conditions that such binding is possible
b. Recovering the variant and/or detecting, or measuring the amount of, the human leptin receptor bound to the variant.

The recovery of b) can be performed by various washes or methods common in the art. The detection or quantification can be performed by any method such as ELISA, chromatography, fluorescence or other methods in the art.

### Diseases to be treated with the variants

As indicated above, the variants herein disclosed are essentially used as means for transporting biologicals through the intestinal barrier. The diseases that are to be treated, using the variants herein disclosed are thus linked to the biological activity of the molecules associated with the variants.

### DESCRIPTION OF THE FIGURES

Figure 1: Alignment of proteins of the Sac7d family.
Figure 2: Verification of binding to hLepR
Figure 3: Competition assay between with human leptin (hLep) and Nanofitins for binding to hLepR
Figure 4: Cross reactivity to mouse Leptin receptor (mLepR) by evaluating binding signal in ELISA
Figure 5: Verification of binding on cellular model expressing hLepR and pig LepR (pLepR)
Figure 6. Verification of expression of pLepR on porcine Jejunum
Figure 7: Improved intestinal barrier crossing in ex vivo assay for Nanofitins from cluster A, compared to a Nanofitin (NF) identified in the screening not belonging to the cluster (F02) and a Nanofitin binding to another target (irrelevant NF)
Figure 8: Conservation of binding properties of Nanofitins alone (F08) or fused to diverse protein sequences (GFP, F08-GFP) or another Nanofitin (F08-NF)
Figure 9: Improved intestinal barrier crossing in ex vivo assay for Nanofitins from cluster A alone (F08) or fused to another Nanofitin (F08-NF) and a Nanofitin binding to another target (irrelevant NF).

### EXAMPLES

### Example 1. Identification of Nanofitin variants that bind to recombinant human Leptin receptor

95 variants (Such variants, based on the Sac7d sequence, are called Nanofitins) binding to Leptin receptor were screened by ELISA from crude bacterial supernatant using immobilized hLepR as a target, showing a high proportion of positive binders (Figure 2). Subsequent sequencing showed strong sequence diversity, with very few repeated sequences. Segregation of the Nanofitins into clusters was performed, based on the homology of the mutations in their binding. Nanofitins for one cluster (cluster "A"

### Example 2. Nanofitins from cluster A bind to LepR on an epitope not overlapping with its natural leptin ligand

Leptin is a 16KDa hormone interacting with leptin receptor and whose main function is to regulate the satiety and energy storage at the hypothalamus location. Leptin interacts more specifically at the second cytokine receptor homology domain, CRH2, of the full Leptin receptor. In addition the interaction with more than one LepR through the Ig-like domain interacting may stabilize binding in these species.

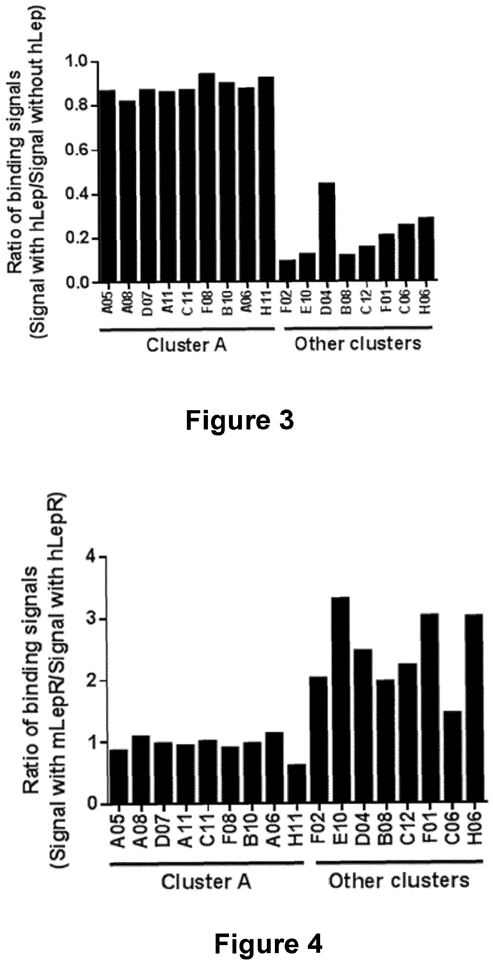

Overlap between the epitopes bound by Nanofitins or the leptin were identified *via* a competition assay, by ELISA, using at least one anti-hLepR Nanofitins from each different sequence clusters (cluster A and others). The results are shown as a ratio of binding, from ELISA signal of the different Nanofitins with the hLepR measured in presence of hLeptin divided by their binding to hLepR measured in absence of hLep (Figure 3). The binding response of the Nanofitins from the cluster A was not affected by the presence of hLep (ratio ~ 1), which suggests that they bind hLepR on an epitope different than the hLep binding site. Nanofitins not included in cluster A showed a reduced binding response in presence of the hLep (ratio < 0.5), suggesting that the hLep is overlapping their epitope.

### Example 3: Nanofitins from cluster A cross react with mouse Leptin receptor and share a similar binding pattern

Cross reactivity between mouse and human LepR was evaluated by ELISA for the Nanofitins found specific to hLepR in cluster A and in other clusters. The cross reactivity was assessed by the ratio of the binding response measured on mLepR divided by the binding response measured on hLepR (Figure 4). Nanofitins from cluster A bind equally to the receptor from both species (ratio ~1) whereas other Nanofitins have increased ELISA signals on mLepR (ratio >1.5), suggesting that the Nanofitins from cluster A bind to an epitope different from Nanofitins of other clusters.

### Example 4. Binding to human and pig leptin receptor on cellular model.

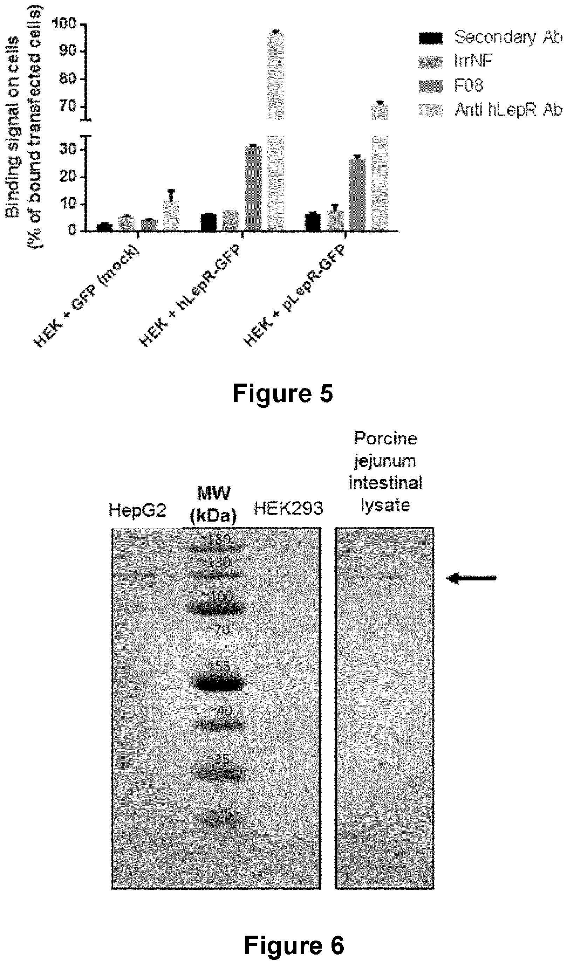

Cells were modified to express either human Leptin receptor (hLepR) or pig Leptin receptor (pLepR) and were used as a model of cell surface LepR. Human (NM_002303.5), and pig (NM_001024587.1) long isoform of the leptin receptor genes were inserted into a plasmid pCNDA3.1+ C-eGFP by Genscript (Piscataway, New Jersey, US), that includes a reporter GFP gene. Plasmids were transfected into HEK293 cells (Manassas, VA, USA). Positively transfected cells were identified by measurement of the GFP fluorescence. The receptor expression was confirmed by flow cytometry using an anti LepR antibody (FAB867R, Bio techne). These cell models were then used to evaluate the ability of the anti-hLepR Nanofitins from cluster A to bind both human and pig Leptin receptors expressed at the cell surface, using the clone F08 as a representative of the cluster A. F08 was found able to bind specifically on hLepR and pLepR expressing cells and not on the mock cell line transfected with an empty vector (Figure 5). This demonstrates that Nanofitins from cluster A are able to bind to the receptor in a cellular context, and that they are able to cross react with pLepR.

### Example 5. Expression of Pig LepR in porcine jejunum.

Functional evaluation of the anti-LepR Nanofitins in porcine intestine models implies that the receptor is effectively expressed in the tissue. Expression of the pLepR in porcine intestine was investigated by western blot (Figure 6). Cells from porcine jejunum intestine were collected and compared to hLepR expressing cells (HepG2) and h/pLepR negative cells (HEK293). Supernatants of cell lysates were run on SDS page gel and h/pLepR was identified by western-blot using an anti-LepR antibody followed by chemiluminescent revelation. Results given in Figure 6 show a band for lysates from HepG2 cells and porcine jejunum at the expected LepR molecular weight (132.5 kDa). As expected, no band could be observed for the condition using the HEK293 lysate, demonstrating the specificity of the anti-LepR staining. Taken together, this data confirmed the expression of LepR in porcine jejunum.

### Example 6. Active transport of anti-LepR Nanofitins through the intestinal barrier is found for the anti-LepR Nanofitins from Cluster A but not for other Nanofitins. including another anti-LepR Nanofitin and an irrelevant Nanofitin.

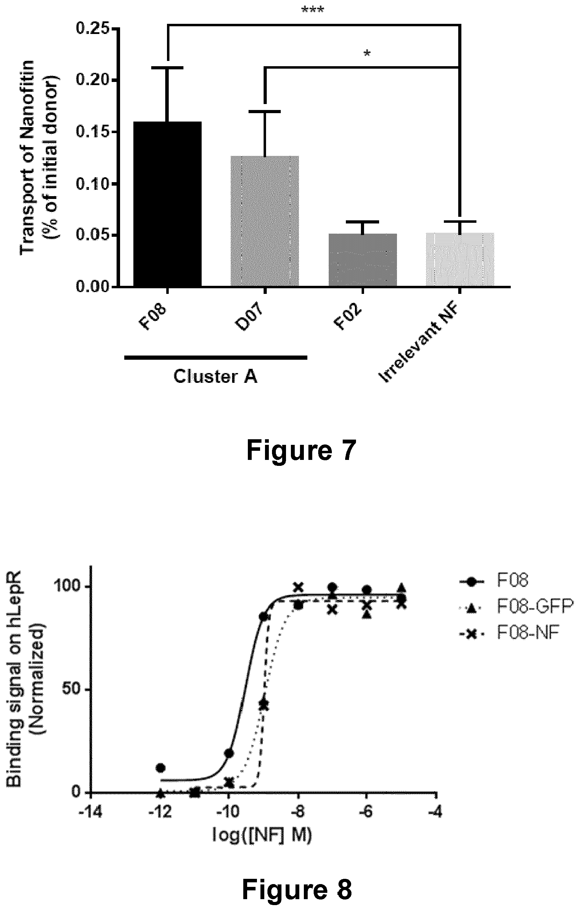

Active translocation through the intestinal barrier of anti-LepR Nanofitins was assessed ex-vivo using porcine intestine tissue introduced in Ussing chambers. Fresh porcine intestine were prepared and inserted in Ussing chamber sliders with an exposed surface area of 1.26 cm² creating a donor compartment (mucosal) and an acceptor compartment (serosal) maintained in KBR buffer at 38°C. Viability and integrity of tissues were followed by electrical resistance (TEER). Fluorescently labelled Nanofitins were introduced in the mucosal compartment and, after 120 minutes, samples from each compartment were analysed to quantify the presence of Nanofitins. The percentage of fluorescently labelled Nanofitins found in acceptor compartment was calculated relatively to the quantity of material applied in the donor compartment, and used as a measure of the transport efficiency across the intestinal barrier (Figure 7). An irrelevant Nanofitin targeting the GFP was used as negative control in the experiment. Percentage of passage in serosal compartment was found similar for the irrelevant Nanofitin and the anti-LepR Nanofitin F02 (=<0.05%), suggesting that such level of passage could be attributed to a passive diffusion. Anti-LepR Nanofitins from the cluster A (F08 and D07) were found to benefit from a higher passage through the intestinal barrier (> 0.05%) than the irrelevant Nanofitin and the other anti-LepR Nanofitin, suggesting the recruitment of an active mechanism for their transport which is highlighted by their significantly higher percentage measured in the serosal compartment. This demonstrates that targeting LepR alone is not sufficient to provide an active delivery through the intestinal barrier and that the Nanofitins from the Cluster A are sharing a common epitope providing a selective advantage toward this property.

### Example 7: Tolerant to fusion at both N- and C-terminus ends.

The binding site of Nanofitins and their N- and C-terminus extremities are located on opposite faces. As a consequence, Nanofitins can be conjugated with a cargo molecule at those extremities without altering their target engagement, which could include the genetic fusion or conjugation to a peptide or a protein. This was demonstrated for the anti-LepR Nanofitin F08. F08 Nanofitin is retaining its functionality regardless of the protein cargo fused at its C-terminus end. This was demonstrated using different proteins in sequence and length. It is to be noted here that a similar property has been observed with the fusion to full length antibodies (WO2019096797) or another Nanofitin. Furthermore, Nanofitins can be expressed either recombinantly in a variety of expression hosts (prokaryotic or eukaryotic) with examples given for *E. coli* and CHO, or by full chemical synthesis. In all the cases explored with the F08 Nanofitin, a fully functional F08 Nanofitin is recovered as demonstrated by ELISA experiment (Figure 8).

### Example 8. Improvement of barrier crossing of protein cargo fused to Nanofitin from Cluster A

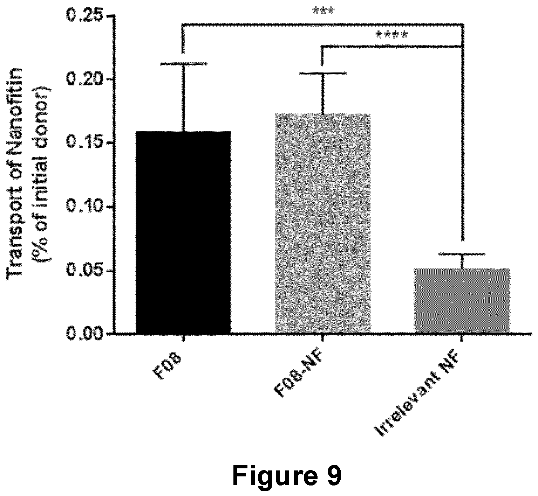

Nanofitins can be fused to other proteins while conserving their binding properties. As a consequence, Nanofitins from cluster A can be conjugated to a cargo protein to be used as a vehicle or trojan-horse to provide active transport of the cargo through the intestinal barrier. This was demonstrated using the ex-vivo experiment setup described in example 6 with F08 fused to a second Nanofitin as cargo (construct F08-NF). The percentage of fluorescently labelled Nanofitin fusions found in acceptor compartment was calculated relatively to the quantity of material applied in the donor compartment, and used as a measure of the transport efficiency across the intestinal barrier (Figure 9). Active transport, highlighted by a percentage in the serosal compartment > 0.05%, was observed for both the Nanofitin F08 alone and its counterpart fused to a cargo protein. Moreover, the percentage of passage in the serosal compartment was very similar for both the Nanofitin F08 alone and its conjugated counterpart, demonstrating that the anti-LepR Nanofitin F08 fully retains its property of active translocation through the intestinal barrier when conjugated to a cargo protein. This result demonstrates that

## Claims

1. A polypeptide comprising a variant of a member of the Sac7d family binding to the human leptin receptor, wherein the variant comprises from 4 to 20 mutated residues in the interface of binding of the member of the Sac7d family to its natural ligand, and wherein said variant comprises W24Y, T33W and A44H mutations with the numbering corresponding to the numbering of Sac7d residues as depicted in SEQ ID NO: 1.

2. The polypeptide of claim 1, further comprising Y8M, K9G, S31G and R42G mutations, with the numbering corresponding to the numbering of Sac7d residues as depicted in SEQ ID NO: 1.

3. The polypeptide of claim 1 or 2, further comprising at least one mutation selected from D16E, N37Q and M57L, with the numbering corresponding to the numbering of Sac7d residues as depicted in SEQ ID NO: 1.

4. The polypeptide of any one of claims 1 to 3, wherein the mutated residues in the interface of binding of the member of the Sac7d family to its natural ligand are selected from the group consisting of V2, K3, K5, K7, Y8, K9, G10, E14, T17, K21, K22, W24, V26, G27, K28, M29, S31, T33, D36, N37, G38, K39, T40, A44, S46, E47, K48, D49, A50 and P51 of Sac7d as depicted in SEQ ID NO: 1.

5. The polypeptide of any one of claim 1 to 4, wherein the member of the Sac7d family is selected from the group consisting of Sac7d from *Sulfolobus acidocaldarius,* Sac7e from *Sulfolobus acidocaldarius,* SSo7d from *Sulfolobus solfataricus,* Ssh7b from *Sulfolobus shibatae,* Ssh7a from *Sulfolobus shibatae,* DBP7 from *Sulfolobus tokodaii,* Sis7a from *Sulfolobus islandicus,* Mse7 from *Metallosphaera sedula,* Mcu7 from *Metallosphaera cuprina,* Aho7a from *Acidianus hospitalis,* Aho7b from *Acidianus hospitalis,* Aho7c from *Acidianus hospitalis* and Sto7 from *Sulfurisphaera tokodaii.*

6. The polypeptide of any one of claims 1 to 5, which comprises SEQ ID NO: 30, SEQ ID NO: 31 SEQ ID NO: 32, SEQ ID NO: 35, SEQ ID NO: 36, SEQ ID NO: 37, SEQ ID NO: 40, SEQ ID NO: 41, SEQ ID NO: 42, SEQ ID NO: 45, SEQ ID NO: 46, SEQ ID NO: 47, SEQ ID NO: 50, SEQ ID NO: 51, SEQ ID NO: 52, SEQ ID NO: 55, SEQ ID NO: 56, SEQ ID NO: 57, SEQ ID NO: 60, SEQ ID NO: 61, SEQ ID NO: 62, SEQ ID NO: 66, SEQ ID NO: 67, SEQ ID NO: 68, SEQ ID NO: 17, SEQ ID NO: 18, SEQ ID NO: 19, SEQ ID NO: 20, SEQ ID NO: 21, SEQ ID NO: 24, SEQ ID NO: 25, SEQ ID NO: 28 or SEQ ID NO: 29.

7. The polypeptide of any one of claims 1 to 5, which comprises SEQ ID NO: 38, SEQ ID NO: 39, SEQ ID NO: 43, SEQ ID NO: 44, SEQ ID NO: 48, SEQ ID NO: 49, SEQ ID NO: 53, SEQ ID NO: 54, SEQ ID NO: 58, SEQ ID NO: 59, SEQ ID NO: 63, SEQ ID NO: 64, SEQ ID NO: 69, SEQ ID NO: 70, or amino acids 1-54 of these sequences.

8. The polypeptide of any one of claims 1 to 7, which consists in the variant of a member of the Sac7d family binding to the human leptin receptor.

9. The polypeptide of any one of claims 1 to 7, wherein the variant of a member of the Sac7d family binding to the human leptin receptor which is conjugated to another polypeptide, in particular another variant of a protein of the Sac7d family.

10. The polypeptide of any one of claims 1 to 7, wherein the variant of a member of the Sac7d family binding to the human leptin receptor which is conjugated to an organic molecule.

11. A nucleic acid molecule coding for the polypeptide of any one of claims 1 to 9.

12. A pharmaceutical composition comprising the polypeptide of any one of claims 1-10, or the nucleic acid of claim 11, and a pharmaceutically acceptable carrier.

13. The polypeptide of any one of claims 1 to 10 or the nucleic acid of claim 11 for use thereof as a medicament.

14. The polypeptide of claim 13, which is bound to a substance having therapeutic activity in a specific disease, for use thereof for the treatment or prevention of this specific disease.
